# EUROPEAN PATENT APPLICATION

(11) **EP 4 059 509 A1**
(43) Date of publication of application: **21.09.2022**
(21) Application number: 19952161.8
(22) Date of filing: 14.11.2019
(51) Int. Cl.: A61K 36/73, A61K 31/7048, A61P 1/16, A23L 33/105, A23L 33/125

(54) **AGRIMONIA EUPATORIA EXTRACT PREPARATION METHOD, AND COMPOSITION FOR IMPROVING LIVER FUNCTIONS OR TREATING LIVER DISEASES, CONTAINING EXTRACT**

(30) Priority: 13.11.2019 KR 20190145293
(71) Applicant: Bionic Trading Corporation, Gyeonggi-do 15588 (KR)
(72) Inventor: KIM, Tae Young, Ansan-si Gyeonggi-do 15463 (KR); MOON, Joo Myung, Ansan-si Gyeonggi-do 15449 (KR); KIM, Hyung Joong, Cheongju-si Chungcheongbuk-do 28123 (KR); KIM, Dong Hyeon, Ansan-si Gyeonggi-do 15628 (KR)
(74) Representative: Nony
(86) International application number: PCT/KR2019/015505
(87) International publication number: WO 2021/095921

(57) **Abstract**

The present invention relates to: an *Agrimonia eupatoria* extract preparation method for promoting the recovery rate and bioavailability of an active ingredient; and a composition for improving liver functions or treating liver diseases, containing the extract. More specifically, the present invention provides an *Agrimonia eupatoria* extract preparation method capable of extracting *Agrimonia eupatoria* by using a solvent in which cyclodextrin is dissolved, thereby increasing the recovery rate and bioavailability of an active ingredient, and provides a health functional food for improving liver functions or an agent for treating liver diseases, the food and the agent containing an *Agrimonia eupatoria* extract and having enhanced efficacy.

## Description

### Technical Field

The present invention relates to a method for preparing an *Agrimonia eupatoria* extract, which increases the recovery of active ingredients and increases bioavailability, and a composition for improving liver function or treating liver disease containing the extract.

More specifically, the present invention provides a method for preparing an *Agrimonia eupatoria* extract, which increases not only the recovery of active ingredients but also bioavailability by extracting *Agrimonia eupatoria* with a solvent containing cyclodextrin dissolved therein, and provides a health functional food for improving liver function or a therapeutic agent for liver disease, which contains an *Agrimonia eupatoria* extract and has increased efficacy.

### Background Art

The liver is the largest organ in our body consisting of more than 300 billion liver cells, weighs 1.2 to 1.5 kg in an adult, and performs an important function in processing and storing substances in the body. The liver receives blood supply from both the hepatic artery and the portal vein, in which the portal vein is a kind of vein filled with various substances absorbed from the stomach and intestines. The nutrients thus absorbed are processed in the liver to become substances necessary for our body, and components harmful to the human body are detoxified. In addition, the liver is an organ that makes bile acids that help digestion, and has immune cells that play an important role in removing bacteria and foreign substances that enter our body.

Examples of components generally known to have a hepatocyte protective effect include glycyrrhizin, catechin, silymarin, and the like. These components have been reported to inhibit the replication of hepatitis virus and have excellent hepatoprotective functions, but they have the disadvantages of low potential for development as therapeutic agents and high usage.

The leaves of *Agrimonia eupatoria* are spherical in shape, have a serrated border, and have irregularly pinnate lamina, with 4 to 5 pairs of main segments. Flowering begins in April and continues until October, and the fruit contains two or rarely one seed. *Agrimonia eupatoria* acts as an anti-inflammatory agent against rheumatoid and muscle inflammation, a decongestant, an anti-allergic agent, a hypoglycemic agent, an anti-diarrheal agent, an antiseptic, an antispasmodic, and a plant treatment against gastroenteritis, and is also used for liver inflammation, prostatitis and benign prostatic hyperplasia, gallbladder disease, hepatic edema, coagulation, oral inflammations (pharyngitis, tonsillitis, inflammatory and bleeding gums, stomatitis), allergic rhinitis, itchy dermatitis, conjunctivitis, etc.

As prior art documents related to the above-described hepatocyte protective effect, Korean Patent No. 10-327762 and pending Korean Patent Application No. 10-2003-0030922 demonstrate that a variety of plants belonging to the genus Agrimonia have inhibitory effects against hepatitis B virus, and disclose pharmaceutical compositions effective for treatment of hepatitis B, which contain an extract of a plant of the genus Agrimonia.

Korea Patent No. 10-0327762 relates to a substance for inhibiting hepatitis B virus surface antigen production isolated from the whole plant of *Agrimonia eupatoria,* a method for isolating the same, and the use thereof as a therapeutic agent for hepatitis/liver cancer. Specifically, it discloses that the substance may inhibit hepatitis B virus surface antigen production, and thus may be used to treat hepatitis and liver cancer caused by hepatitis B virus infection. Korea Patent No. 10-0557015 discloses the prevention or treatment of liver disease using a pharmaceutically effective amount of an *Agrimonia eupatoria* extract. In addition, it discloses that the *Agrimonia eupatoria* extract exhibits a liver function protective effect by restoring AST and ALT levels, which are representative liver injury indexes (Korean Journal of Pharmacognosy 37.1 (2006): 28.). As a patent related to liver function protection, Korea Patent Application Publication No. 2011-0010601 relates to *Agrimonia eupatoria*-derived phenolic acid- and flavonoid-based compounds or salts thereof, which exhibit a hepatotoxicity preventive or therapeutic effect without side effects, and a composition for liver protection containing the same, and it discloses that *Agrimonia eupatoria*-derived phenolic acid-based compounds and flavonoid-based compounds can increase the cell viability of HepG2 cells, a human-derived hepatocyte cell line having toxicity induced by carbon tetrachloride, reduce toxicity to the cancer cells, and thus have hepatoprotective efficacy without side effects.

Active ingredients involved in the hepatocyte protective activity of *Agrimonia eupatoria* are disclosed in Korean Journal of Pharmacognosy 37.1 (2006): 28, and include nine effective compounds, including isoquercitrin, quercitrin, luteolin-7-O-glucuronide, astragalin, rutin, and the like. Among them, isoquercitrin is contained in *Houttunynica cordata, Stewartia koreana,* cucumber, etc., which exhibit a hepatoprotective effect, and an animal experiment using white rats also indicated that isoquercitrin exhibits a liver function protective effect by restoring AST and ALT levels which are liver injury indexes (Oncotarget, 2017, Vol. 8, (No. 60), pp: 101545-101559). It is known that qurecitrin and luteolin-7-O-glucuronide, which are isomers of isoquercitrin, also exhibit an excellent hepatoprotective effect, and they have been reported to have an excellent hepatoprotective effect in prior patents and literature.

Cyclodextrin is an oligosaccharide containing glucose molecules linked together to form a cyclic ring, and is internally hydrophobic and externally hydrophilic. For this reason, cyclodextrin is frequently used to form inclusion complexes with hydrophobic substances, rather than functioning as an oligosaccharide, to increase solubility, bioavailability, and stability of the hydrophobic substances. In recent years, a technique of improving inclusion complex-forming ability and stabilization ability by modifying the structures of linked glucose structures to enhance inclusion complex-forming properties has also been used.

Cyclodextrins are classified into alpha, beta, and gamma cyclodextrins depending on the number of glucose molecules forming a cyclic ring, but compounds capable of forming inclusion complexes are limited according to their three-dimensional size. Thus, it is not easy to select the optimal cyclodextrin for forming inclusion complexes with natural products containing various compounds.

Meanwhile, the increase in bioavailability using an inclusion agent is widely known in prior patents, etc., but in order to form an inclusion complex with an extract, the extract is re-dissolved and adjusted to an appropriate pH, and then the operation of heating and stabilizing needs to be repeated, which significantly increases the process time and cost.

An *Agrimonia eupatoria* extract showing hepatoprotective efficacy is obtained by simple water extraction or a simple solvent extraction method using a mixture of water and alcohol. However, since many of the substances exhibiting efficacy are insoluble in water, this method has a disadvantage in that it is necessary to excessively increase the temperature or increase the proportion of solvents other than water in order to recover these substances. In addition, in the case of a simple extract, poorly water-soluble components may aggregate during cooling after heat extraction of poorly soluble components or may be adsorbed to the extraction residue.

It has been found that, when inclusion extraction is performed after cyclodextrin is dissolved in water or a C₁-C₄ lower alcohol mixture solution at a certain ratio, the formation of inclusion complexes with indicator active ingredients is achieved, the bioavailability of the extract is increased, and the *in vivo* efficacy of the extract is also increased at the same dose compared to an existing extract. In addition, in the case of general extraction, several active ingredients may adhere again to the extraction residue or there may be a decrease in extraction efficiency due to high concentrations of the active ingredients in the extract, but according to the present invention, it has been found that the active ingredients have increased solubility by forming inclusion complexes with cyclodextrin during extraction, resulting in an increase in the extraction yield, thereby completing the present invention.

### DISCLOSURE

### Technical Problem

An object of the present invention is to provide a method for preparing an *Agrimonia eupatoria* extract, which improves the yield through inclusion extraction, increases the recovery of active ingredients for liver function improvement, and increases the bioavailability of the extract.

Another object of the present invention is to provide a food composition or pharmaceutical composition containing an *Agrimonia eupatoria* inclusion extract, in which active ingredients isolated from the *Agrimonia eupatoria* inclusion extract and the *Agrimonia eupatoria* inclusion extract containing the active ingredients have increased efficacy in terms of liver function evaluation indicators such as ALT, AST, and ALP levels, and protective effects against hepatocellular injury, determined through liver histopathological analysis.

### Technical Solution

According to one aspect of the present invention, the present invention provides a method for preparing an *Agrimonia eupatoria* extract comprising step a) of obtaining an extract from *Agrimonia eupatoria* using an organic solvent containing cyclodextrin.

Specifically, the step of obtaining comprises: step a-1) of preparing an extraction solvent by adding cyclodextrin to the organic solvent; and step a-2) of obtaining the extract by adding *Agrimonia eupatoria* to the extraction solvent.

Step a-1) may comprise preparing the extraction solvent by heating at 20 to 100°C for 3 to 8 hours after adding cyclodextrin to the organic solvent.

As the organic solvent, C₁ to C4 lower alcohols known in the art may be used without limitation, and among them, at least one solvent may be used. An example of the organic solvent may be at least one selected from the group consisting of methanol, ethanol, n-propanol, iso-propanol, n-butanol, iso-butanol, 2-butanol and 3-butanol. Preferably, it may be ethanol or fermented alcohol.

The cyclodextrin may be at least one selected from the group consisting of alpha-cyclodextrin, beta-cyclodextrin and gamma-cyclodextrin. Preferably, it may be gamma-cyclodextrin. In this case, the cyclodextrin may be used in an amount of 0.001 to 50 parts by weight, preferably 0.1 to 40 parts by weight, more preferably 0.5 to 30 parts by weight, based on the total weight of *Agrimonia eupatoria* added in step a-2).

The dissolution temperature of the cyclodextrin may be 20 to 100°C, preferably 60 to 90°C. In this case, in order to increase the solubility of the cyclodextrin in the organic solvent, pH may be appropriately adjusted.

Step a-2) may comprise obtaining the extract by extraction at 20 to 100°C for 1 to 40 hours after adding *Agrimonia eupatoria* to the extraction solvent.

In the extraction conditions, the extraction temperature may be 20 to 100°C, preferably 40 to 90°C lower than the boiling point of alcohol or water, more preferably 60 to 80°C. The extraction time may change depending on the extraction temperature, and may be 1 to 40 hours, preferably 2 to 24 hours, more preferably 3 to 10 hours.

To obtain the extract, the roots, stems and leaves of *Agrimonia eupatoria,* and the whole plant including the same may be used without limitation. Preferably, the whole plant of *Agrimonia eupatoria* may be used. At this time, *Agrimonia eupatoria* may be in a raw material state, a dry state, or a mixed state thereof, and may be in a finely pulverized state so that active ingredients may be efficiently extracted. *Agrimonia eupatoria* may be used in an amount of 0.01 to 50 parts by weight, preferably 0.05 to 30 parts by weight, more preferably 0.1 to 10 parts by weight, based on the total weight of the extraction solvent.

The extract may contain active ingredients isolated from *Agrimonia eupatoria,* and in particular, may contain sparingly soluble components isolated from *Agrimonia eupatoria.* Examples of such active ingredients include isoquercitrin, quercitrin, luteolin-7-O-glucuronide, astragalin, rutin, apigenin-7-O-β-D-glucuronide, etc. Preferably, the active ingredients include at least one selected from the group consisting of isoquercitrin, quercitrin, luteolin-7-O-glucuronide, and astragalin. In this case, the extract may contain 0.1 to 5% by weight of luteolin-7-O-glucuronide, which is an indicator active ingredient and forms an inclusion complex.

The method may further comprise, after step a), step b) of filtering the extract.

Step b) may be performed using any filtration method known in the art without limitation, and may be repeated one or more times to remove impurities from the extract. As an example of the filtration method, a filter press, a centrifuge, filter paper, etc. may be used. In this case, it is possible to separate the extract into the filtrate and the filtration residue, which is a solid remaining after filtration.

After step b) of filtering, it is possible to further obtain an extract by reusing the filtration residue separated from the extract. Specifically, the method may further comprise step b') of obtaining an extract from the filtration residue using an organic solvent containing cyclodextrin. In this case, the cyclodextrin and the organic solvent are as described above, and the step of obtaining the extract is also as described above. This step b') may be repeated one or more times. The extract (secondary extract) obtained from the filtration residue may be mixed with the filtrate (primary extract) of step b') and used in a subsequent process.

The method may further comprise, after step a), step c) of concentrating the extract. Alternatively, after step b) or step b'), step c) of concentrating the extract may be performed.

Step c) may be performed using a concentration method known in the art without limitation to evaporate the extraction solvent and increase the concentration of solids. An example of the concentration method may be concentration under reduced pressure, ultrafiltration, centrifugation, or the like.

The method may further comprise, after step a), step d) of drying the extract. Alternatively, after step c), step d) of drying the extract may be performed.

Step d) may be performed using any drying method known in the art without limitation, and the extract may be powdered through the drying process. An example of the drying method may be freeze drying, vacuum drying, spray drying, hot air drying, or the like. Preferably, the water content of the powder may not exceed 10%.

The *Agrimonia eupatoria* extract prepared by the above method may have an extraction yield of 20% or more, is characterized by increased yield and bioavailability compared to an *Agrimonia eupatoria* extract prepared by a conventional method, and may exhibit a liver function improvement effect by improving ALT and AST levels, which are liver function injury indicators, even at a lower dose.

Therefore, the *Agrimonia eupatoria* extract may be used as a food composition for improving liver function or a pharmaceutical composition for treating liver disease. In this case, the active ingredient content of the extract in the composition may be 0.01 to 20% (dry weight basis), preferably 0.03 to 10% (dry weight basis). For example, the content of luteolin-7-0-glucuronide as an indicator active ingredient may be 0.01 to 20% (dry weight basis), preferably 0.03 to 10% (dry weight basis).

The dose of the extract usable in the food composition or pharmaceutical composition may be 20 to 1,000 mg/day for adults, preferably 40 to 500 mg/day, and more preferably 80 to 250 mg/day, in view of economic efficiency. The *Agrimonia eupatoria* extract in the food composition or pharmaceutical composition may be contained in an amount of 0.001 to 100 wt%, preferably 0.05 to 90.0 wt%, based on the total weight of the final composition.

The food composition for improving liver function may be provided in any formulation suitable for food additives or functional foods, and may be, for example, in the form of solutions, emulsions, viscous mixtures, powders, granules, tablets, capsules, or the like. In this case, the food composition may contain various bases and/or additives necessary and appropriate for formulation within the range that does not impair the main effect of the present invention. In addition, the food composition may further contain additives such as flavorings, colorants, sterilants, antioxidants, preservatives, moisturizing agents, thickeners, inorganic salts, and emulsifiers within the range that does not impair the effect of the present invention. The contents of these additives may be selected depending on the formulation or purpose of use, within a range that does not impair the purpose and effect of the present invention. For example, the contents of the additives may be 0.01 to 5 wt%, more specifically 0.1 to 3 wt%, based on the total weight of the food composition.

The pharmaceutical composition for the treatment of liver disease may be provided in any formulation suitable for pharmaceuticals, and may be, for example, in the form of a solid, semi-solid or liquid for oral or parenteral administration. Examples of formulations for oral administration include tablets, pills, granules, capsules, powders, fine granules, powders, emulsions, syrups, pellets, and the like. Examples of formulations for parenteral administration include injections, drops, ointments, lotions, sprays, suspensions, emulsions, suppositories, and the like. In this case, the pharmaceutical composition may contain various bases and/or additives necessary and appropriate for formulation within the range that does not impair the main effect of the present invention. In addition, the pharmaceutical composition may further contain additives such as carriers, surfactants, excipients, colorants, spices, preservatives, stabilizers, buffers, and suspending agents within the range that does not impair the effect of the present invention. This pharmaceutical composition may be administered orally, parenterally, rectally, topically, transdermally, intravenously, intramuscularly, intraperitoneally, subcutaneously or the like. The active ingredient of the pharmaceutical composition may vary depending on the age, sex, weight, pathological condition and severity of the subject to whom the composition is to be administered, the route of administration, or the judgment of the prescriber. For example, the daily dose thereof may be 0.01 to 100 mg/kg, more specifically 0.1 to 50 mg/kg.

### Advantageous Effects

According to the present invention, it is possible to prepare an *Agrimonia eupatoria* extract with an increased recovery rate of active ingredient compounds effective for improving liver function. In addition, when the *Agrimonia eupatoria* extract is prepared by the novel method, it is possible to economically provide an extract containing active ingredients as inclusion complexes by inclusion without the need to introduce an additional process for forming inclusion complexes with cyclodextrin. In addition, it has been confirmed that the extract thus prepared has increased bioavailability, and thus improves liver function evaluation indicators such as ALT, AST, and ALP at a lower dose, and exhibits a protective effect against liver cell injury as demonstrated by liver histopathological analysis, suggesting that the extract may be effectively used for improving liver function and preventing and treating liver disease.

### Brief Description of Drawings

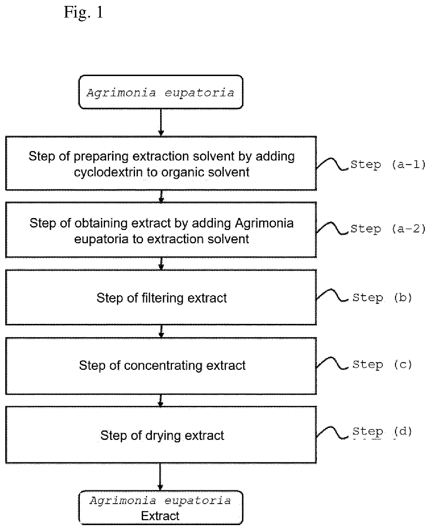

FIG. 1 is a process diagram showing a novel method for preparing an *Agrimonia eupatoria* extract according to one embodiment of the present invention, which increases the recovery of active ingredients for improving liver function.
FIG. 2 shows the results of testing Caco-2 cell uptake of luteolin-7-O-glucuronide, which is an indicator active ingredient of an extract obtained using each type of cyclodextrin as an inclusion agent according to Preparation Example 1 of the present invention.
FIG. 3 shows the results of recovering precipitates and solids depending on each type of cyclodextrin according to Preparation Example 1 of the present invention. (a) depicts photographs showing the results of visually comparing precipitates after standing in 0.5% aqueous solution for 2 hours, and (b) is a graph showing the results of calculating the recovery of solids, dried after centrifuging each supernatant, as a percentage of the weight of a sample added.
FIG. 4 shows the results of HPLC measurement of the increase in the degree of hydration for fat-soluble substances of the extract according to Preparation Example 1 of the present invention.
FIG. 5 shows the results of measuring yield under various cyclodextrin concentration and inclusion extraction temperature conditions according to Preparation Example 2 of the present invention.
FIG. 6 shows the results of testing Caco-2 cell uptake of luteolin-7-O-glucuronide, which is an indicator active ingredient of an inclusion extract, under various cyclodextrin concentration and inclusion extraction temperature conditions according to Preparation Example 2 of the present invention.
FIG. 7 shows the results under various cyclodextrin concentration and inclusion extraction temperature conditions according to Preparation Example 2 of the present invention. (a) depicts photographs showing the results of visually comparing precipitates after standing in a 0.5% aqueous solution for 2 hours, and (b) is a graph showing the results of calculating the recovery of solids as a percentage of the weight of a sample added, after drying of the solids obtained by centrifugation of each supernatant.
FIG. 8 shows the results of performing the measurement of AST and ALT, which are liver injury indicators, for an *Agrimonia eupatoria* extract according to Experimental Example 1 of the present invention.
FIG. 9 depicts photographs showing the results of analyzing the liver injury protective effect of an *Agrimonia eupatoria extract* according to Experimental Example 1 of the present invention, and shows the results of performing histopathological examination of the white rat liver.

### Mode for Invention

Hereinafter, the present invention will be described in more detail with reference to the accompanying drawings. However, the following examples are described for the purpose of illustrating the present invention, and the scope of the present invention is not to be construed as being limited by the following examples.

### Preparation Example 1. Selection of cyclodextrin type

In order to prepare an *Agrimonia eupatoria* inclusion extract using each type of cyclodextrin, *Agrimonia eupatoria* leaves purchased through Jiyu Boncho Co., Ltd. were used.

Specifically, foreign substances were removed from the leaves of *Agrimonia eupatoria,* and each of dextrin as a control and alpha-cyclodextrin, beta-cyclodextrin, and gamma-cyclodextrin as treatment groups was added to 50% fermented alcohol in an amount of 0.13% relative to the liquid amount (2% relative to raw material), thus preparing solutions. A 15-fold weight of each prepared solution was added to 50 g of the *Agrimonia eupatoria* sample, and each mixture was stirred and extracted at 300 rpm at 80°C for 5 hours, and then filtered through filter paper to obtain each primary extract. A 14-fold weight of each fresh dextrin or cyclodextrin solution was added to the filtration residue, and each mixture was stirred and extracted at 300 rpm at 80°C for 5 hours, and filtered through filter paper, and the filtrate was combined with the primary extract, concentrated under reduced pressure at 60°C, and then freeze-dried, thus preparing final extracts.

### 1-1. Measurement of yield and content

The extraction yield was determined by calculating the weight of the freeze-dried extract as a percentage of the weight of the sample used for preparing the extract.

To measure the content of the active ingredient luteolin-7-O-glucuronide in each of the prepared *Agrimonia eupatoria* extract powder (control group) and inclusion extract powder (treatment group), high-performance liquid chromatography (Infinity 1260, Agilent, USA) with X Select HSS C18 Column (Waters, 4.6^{∗}250 mm, 5 µm particle size) was used, and the detection spectrum was measured at 350 nm UV. Distilled water containing 0.1% phosphoric acid was used for mobile phase A, and 100% ACN was used for mobile phase B. The conditions of the mobile phases with time were as follows. Mobile phase A was kept at 90% at 0 to 20 min, 90% to 88% at 20 to 35 min, 88% at 35 to 60 min, 88% to 70% at 60 to 70 min, 70% to 20% at 70 to 72 min, 20% at 72 to 75 min, 20% to 90% at 75 to 76 min, and 90% at 76 to 90 min. The low rate was 1.6 ml/min, and the sample amount was 5 µl. For standard solution preparation, an active ingredient and 20 mg of a precursor standard were placed in a 20-ml flask, and dissolved in DMSO to make a standard solution, and diluted at each concentration. The standard solution was analyzed, and then a calibration curve was prepared to measure the content of each active ingredient. To prepare a sample solution, 500 mg of the sample was placed in a 50-ml flask, dissolved in 70% MeOH, shaken with ultrasonic waves, and filtered through a 0.45 µm filter, followed by measuring the content of the active ingredient luteolin-7-O-glucuronide. The results are shown in Table 1 below.

**[Table 1]**

| | Control | α-CD | β-CD | γ-CD |
|---|---|---|---|---|
| Actual yield (%) | 22.7 | 23.2 | 22.0 | 24.6 |
| Active ingredient content (mg/g) | 6.5 | 6.7 | 6.1 | 7.1 |
| Yield (yield X content) | 147.4 | 155.2 | 134.0 | 174.9 |

As shown in Table 1 above, it was conformed that, when extraction with the solvent containing gamma-cyclodextrin was performed, the yield and content tended to increase compared to those of the control, and in particular, the yield increased by 18% compared to that of the control.

### 1-2. Bioavailability measurement

Cell uptake of the *Agrimonia eupatoria* inclusion extract was measured using Caco-2 cells.

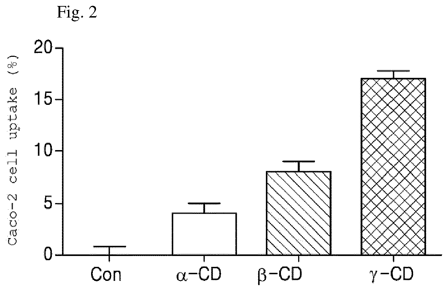

First, Caco-2 cells were seeded into Trans-wells and cultured using MEM medium containing 20% FBS, and then culture was performed while replacing the MEM medium until a membrane was formed. The *Agrimonia eupatoria* extract dissolved in medium was added to insert wells, and after 30 minutes, the medium of the basolateral well was collected. Uptake evaluation was performed by measuring the value transferred from the insert well to the basolateral well through HPLC analysis of each collected medium. The results are shown in FIG. 2.

As shown in FIG. 2, it was confirmed that all the experimental groups showed higher Caco-2 cell uptake than the control group, and in particular, the uptake of the group extracted using gamma-cyclodextrin as an inclusion agent was about 18%, which was about 3 times higher than that of the experimental group extracted using alpha- or beta-cyclodextrin as an inclusion agent.

### 1-3. Water solubility measurement

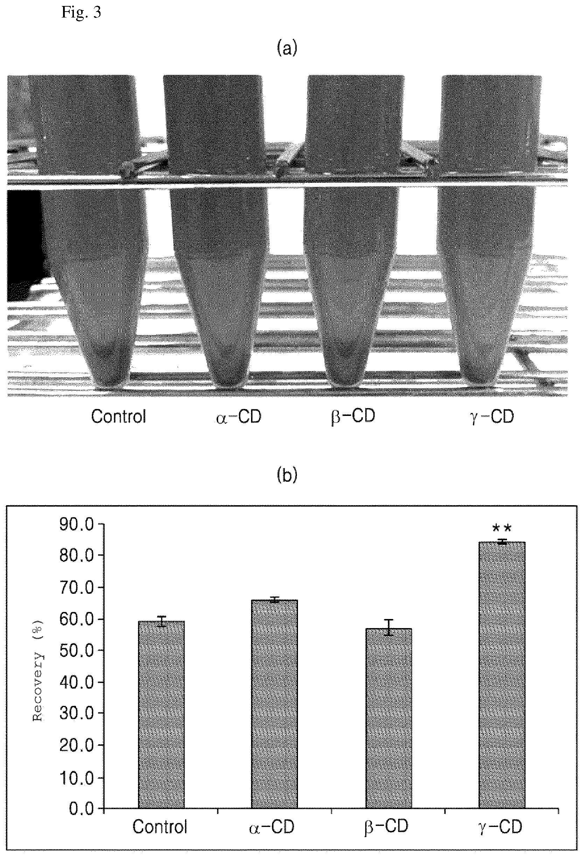

1 g of the *Agrimonia eupatoria* inclusion extract prepared using each type of cyclodextrin was dissolved in 50 mL of purified water and centrifuged for 20 minutes at 4,000 rpm at room temperature. A supernatant of the centrifuged solution was collected and dried using a freeze dryer. The weight of the dried sample was measured, and the recovery rate was determined by calculating the weight of the sample, obtained by drying the supernatant, as a percentage of the weight of the sample added. The results are shown in FIG. 3.

As shown in FIG. 3, it was confirmed that the amount of the precipitate in the group extracted using gamma-cyclodextrin as an inclusion agent was smaller than that in the control group, and the recovery rate of solids in the group was significantly higher than that in the control group.

### 1-4. HPLC measurement

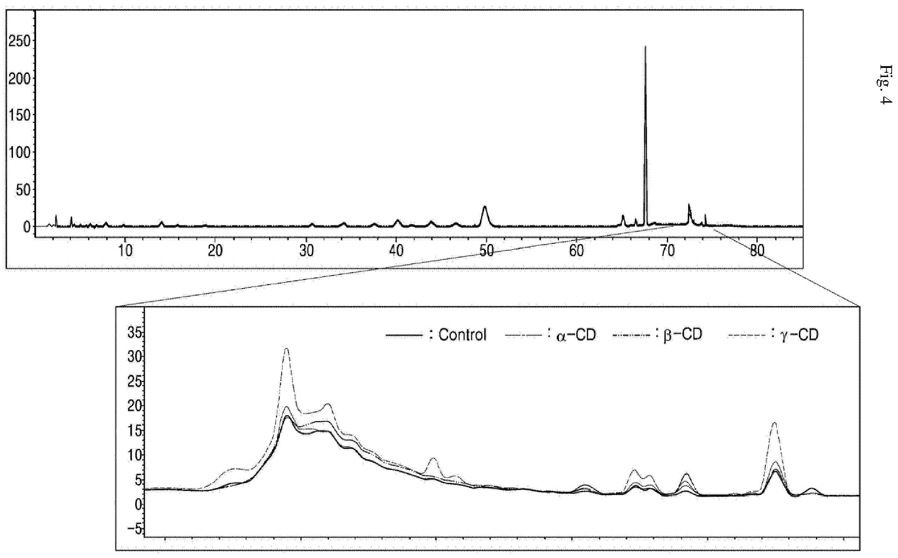

To measure the water solubility of the fat-soluble substances of the prepared *Agrimonia eupatoria* extract powder and inclusion extract powder, analysis was performed in the same manner as in Preparation Example 1-1 using high-performance liquid chromatography (Infinity 1260, Agilent, USA). The results are shown in FIG. 4.

As shown in FIG. 4, it was confirmed that the water solubility of the fat-soluble component of the group extracted using gamma-cyclodextrin as an inclusion agent increased compared to that of the control group.

### 1-5. Analysis of content of indicator active ingredient forming inclusion complex

Many literatures and patents have already demonstrated that cyclodextrin inclusion complexes maintain their water solubility even at low pH compared to non-inclusion complexes. Thus, in order to measure the content of the indicator active ingredient luteolin-7-O-glucuronide forming a inclusion complex in each of the prepared *Agrimonia eupatoria* extract powder (control group) and the inclusion extract powder (treatment group), 100 g of each inclusion extract powder was dissolved in 1 L of purified water, and then adjusted to pH 2.7 by addition of 2N HCl, and centrifuged at 4,000 rpm at room temperature for 20 minutes, and the precipitate and the supernatant were separately collected. The supernatant was concentrated and freeze-dried, and the precipitate was diluted with purified water and freeze-dried. Each dried sample was weighed and the yield was calculated. In the yield calculation method, the yield was determined by calculating the weight of each dried sample, obtained from each of the supernatant and the precipitate, as a percentage of the weight of the initially added sample.

The content of the indicator active ingredient luteolin-7-O-glucuronide in the dry powder obtained from each of the obtained supernatant and precipitate was analyzed in the same manner as in Preparation Example 1-1 using high-performance liquid chromatography (Infinity 1260, Agilent, USA). The results are shown in Table 2 below.

**[Table 2]**

| | | Control | α-CD | β-CD | γ-CD |
|---|---|---|---|---|---|
| Precipitate (non-inclusion complex) | Total yield (g) of precipitate | 71 | 59 | 62 | 32 |
| | Luteolin-7-O-glucuronide content (mg/g) in precipitate | 7.7 | 7.9 | 6.5 | 6.5 |
| | Yield of luteolin-7-O-glucuronide in precipitate | 545.6 | 466.9 | 404.4 | 206.6 |
| Supernatant (inclusion complex) | Supernatant yield (g) | 29 | 41 | 38 | 68 |
| | Luteolin-7-O-glucuronide content in supernatant (mg/g) | 3.6 | 5.3 | 4.9 | 8.3 |
| | Yield of luteolin-7-O-glucuronide in supernatant | 104.4 | 217.3 | 186.2 | 564.4 |
| Inclusion rate calculation | Inclusion rate (supernatant luteolin-7-O-glucuronide yield / total yield ^{∗} 100) | 16.1 | 31.8 | 31.5 | 73.2 |

As shown in Table 2 above, it was confirmed that the inclusion rate of the extract obtained using gamma-cyclodextrin as an inclusion agent was more than 5 times higher than that of the control, and the content of the indicator active ingredient (luteolin-7-O-glucuronide) forming an inclusion complex was also more than doubled.

Through the above experiments, it was confirmed that the use of the gamma-cyclodextrin solution could effectively extract the active ingredient of *Agrimonia eupatoria.* Thus, gamma-cyclodextrin was used in subsequent experiments.

### Preparation Example 2. Determination of cyclodextrin concentration and inclusion extraction temperature

The following experiment was performed to select the concentration of gamma-cyclodextrin and the inclusion extraction temperature. The amounts of gamma-cyclodextrin used were 0%, 2%, and 4% of the weight of the raw material (*Agrimonia eupatoria* leaves), and the inclusion extraction temperatures were 60°C, 70°C, and 80°C.

Specifically, foreign substances were removed from the leaves of *Agrimonia eupatoria,* and a solution was prepared by adding dextrin and/or gamma-cyclodextrin (see Table 3 below) to 50% fermented alcohol. 50 g of the *Agrimonia eupatoria* sample was added to each of three bottles, and a 15-fold weight of the prepared solution was added to the sample, and the mixture was stirred and extracted for 5 hours at 300 rpm at each of 60°C, 70°C and 80°C, and then filtered through filter paper to obtain a primary extract. A 14-fold weight of the same fresh solution was added to the filtration residue, and the mixture was stirred and extracted at 300 rpm for 5 hours at the same temperature as the first extraction temperature (each of 60°C, 70°C, and 80°C) in each bottle, and filtered through filter paper, and then the filtrate was combined with the primary extract, concentrated under reduced pressure at 60°C, and then freeze-dried, thus preparing final extracts.

### 2-1. Measurement of yield and content

The extraction yield was determined by calculating the weight of the freeze-dried sample as a percentage of the weight of the sample used to prepare the extract.

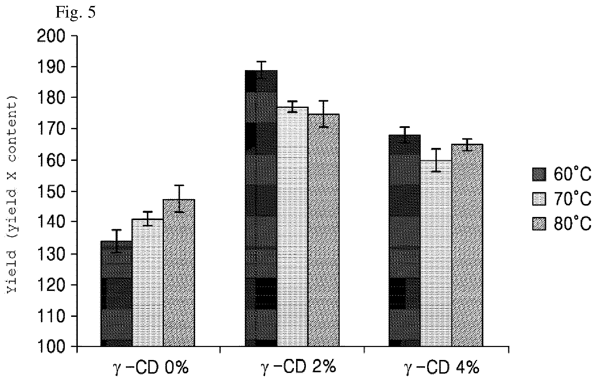

To measure the content of the active ingredient luteolin-7-O-glucuronide in each of the prepared *Agrimonia eupatoria* extract powder (control) and inclusion powder (treatment group), analysis was performed in the same manner as in Preparation Example 1-1 using high-performance liquid chromatography (Infinity 1260, Agilent, USA). The results are shown in Table 3 below and FIG. 5.

**[Table 3]**

| | | γ-CD 0% | γ-CD 2% | γ-CD 4% |
|---|---|---|---|---|
| Solution | Dextrin^{∗} | 0.26% | 0.13% | - |
| | Gamma-cyclodextrin* | - | 0.13% | 0.26% |
| Yield (yield X content) | 60°C^{∗∗} | 134.1 | 188.8 | 168.5 |
| | 70°C^{∗∗} | 141.2 | 177.0 | 160.9 |
| | 80°C^{∗∗} | 147.7 | 174.8 | 165.2 |
| ^{∗} Amount of dextrin and/or gamma cyclodextrin relative to liquid amount | | | | |
| ^{∗∗} Inclusion extraction temperature | | | | |

As shown in Table 3 above and FIG. 5, it was confirmed that the highest yield was obtained when extraction was performed at 60 °C and at a gamma-cyclodextrin concentration of 2%.

### 2-2. Measurement of bioavailability

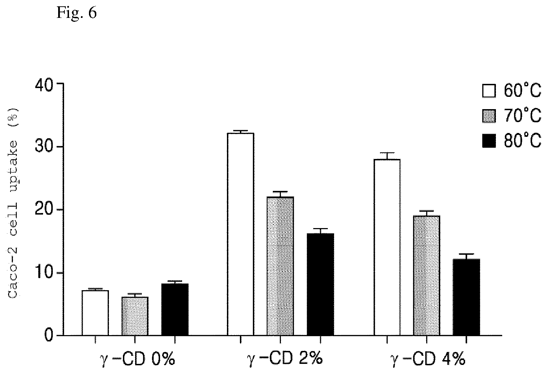

Cell uptake of the *Agrimonia eupatoria* inclusion extract was measured in the same manner as in Preparation Example 1-2 using Caco-2 cells. The results are shown in FIG. 6.

As shown in FIG. 6, it was confirmed that the bioavailability was the highest when extraction was performed at 60°C and at a gamma-cyclodextrin concentration of 2%.

### 2-3. Water solubility measurement

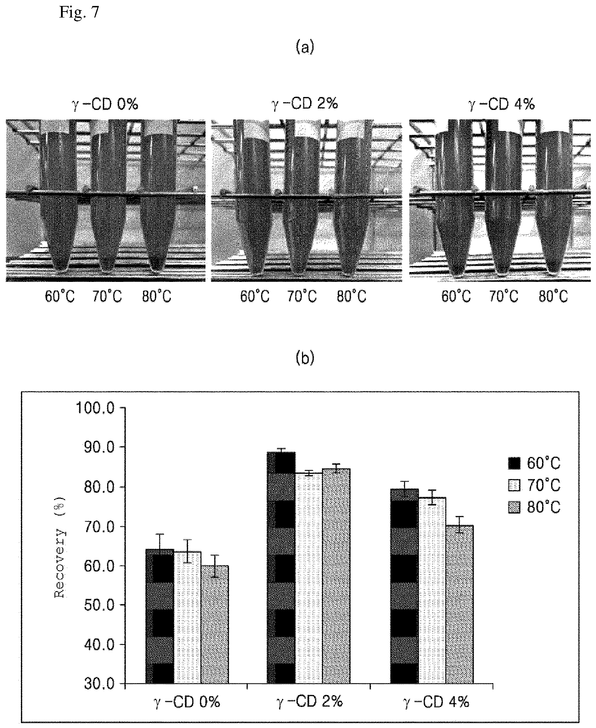

The recovery rate was calculated in the same manner as in Preparation Example 1-3 using 2.5 g of each of the *Agrimonia eupatoria* inclusion extracts obtained under various inclusion conditions. The results are shown in FIG. 7.

As shown in FIG. 7, it was confirmed that the solids recovery rate was the highest when extraction was performed at 60°C and at a gamma-cyclodextrin concentration of 2%.

Through the above experiments, the gamma-cyclodextrin concentration was set to 2% of the raw material, the inclusion extraction temperature was set to 60°C, and subsequent experiments were conducted.

### Example 1. Preparation of Agrimonia eupatoria inclusion extract

In order to prepare an optimized *Agrimonia eupatoria* inclusion extract, *Agrimonia eupatoria* leaves purchased through Jiyu Boncho Co., Ltd. were used.

Specifically, foreign substances were removed from the leaves of *Agrimonia eupatoria,* and gamma-cyclodextrin was added to 50% fermented alcohol in an amount of 0.13% relative to the liquid amount (2% relative to raw material), thus preparing a gamma-cyclodextrin solution. A 15-fold weight of the prepared gamma-cyclodextrin solution was added to 50 g of the *Agrimonia eupatoria* sample, and the mixture was stirred and extracted at 300 rpm at 60°C for 5 hours, and then filtered through filter paper to obtain a primary extract. A 14-fold weight of a fresh gamma-cyclodextrin solution was added to the filtration residue, and the mixture was stirred and extracted at 300 rpm at 60°C for 5 hours, and filtered through filter paper, and the filtrate was combined with the primary extract, concentrated under reduced pressure at 60°C, and then freeze-dried, thus preparing a final extract. The extraction yield was 25.93%, which was determined by calculating the weight of the freeze-dried sample as a percentage of the weight of the sample used to prepare the extract.

### Comparative Example 1. Preparation of Agrimonia eupatoria extract

In order to prepare an *Agrimonia eupatoria* extract, *Agrimonia eupatoria* leaves purchased through Jiyu Boncho Co., Ltd. were used.

Specifically, foreign substances were removed from the leaves of *Agrimonia eupatoria,* and dextrin was added to 50% fermented alcohol in an amount of 0.13% relative to the liquid amount (2% relative to raw material), thus preparing a dextrin solution. A 15-fold weight of the prepared dextrin solution was added to 50 g of the *Agrimonia eupatoria* sample, and the mixture was stirred and extracted at 300 rpm at 80°C for 5 hours, and then filtered through filter paper to obtain a primary extract. A 14-fold weight of a fresh dextrin solution was added to the filtration residue, and the mixture was stirred and extracted at 300 rpm at 80°C for 5 hours, and filtered through filter paper, and the filtrate was combined with the primary extract, concentrated under reduced pressure at 60°C, and then freeze-dried, thus preparing a final extract. The extraction yield was 22.68%, which was determined by calculating the weight of the freeze-dried sample as a percentage of the weight of the sample used to prepare the extract.

### Experimental Example 1. Analysis of liver injury protective effect of Agrimonia eupatoria extract

As experimental animals, 6-7 week-old Sprague-Dawley white rats were used and bred at room temperature of 22±2°C. For the experiment, white rats having similar weights were divided into 4 groups: a positive control group not treated with carbon tetrachloride (CC14); a negative control group treated with CC14 alone; a group treated with 50 mg/kg of the *Agrimonia eupatoria* extract powder of Comparative Example 1; and a group treated with 50 mg/kg of the *Agrimonia eupatoria* inclusion extract powder of Example 1. Experimental diet was provided by free drinking water, and the intake by each group was recorded at a fixed time once a day.

In order to examine the acute hepatotoxicity preventive effect and antioxidant effect of each of the *Agrimonia eupatoria* extract powder and the inclusion extract powder, the experimental diet was supplied every week for one week (see Korean Patent Application Publication No. 10-2012-0108797), and CC14 was administered at the last week. Then, the rats were fasted for 24 hours and then sacrificed. For administration of CC14, 10% CC14 was mixed with olive oil at a ratio of 1:1 and administered at a dose of 1 ml/kg. After fasting, blood was collected from the abdominal aorta, and the collected blood was immediately centrifuged at 3,000 rpm for 15 minutes, and serum was separated therefrom and used as an analysis sample. In addition, the liver, kidney, heart, and spleen were extracted immediately after blood collection, rinsed in physiological saline, and weighed after removal of water from the surfaces thereof, and then stored frozen at -70°C.

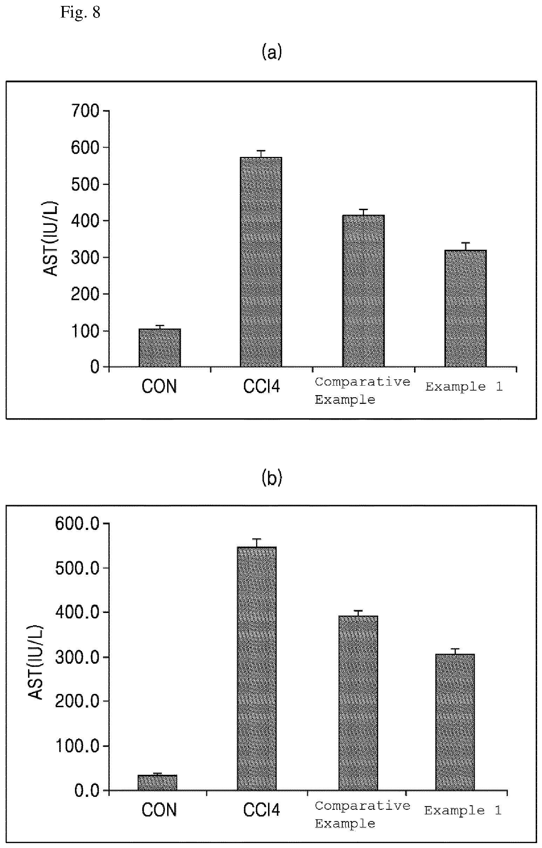

For AST (aspartate aminotransferase) and ALT (alanine aminotransferase) measurement in serum, blood collected from the abdominal aorta was immediately centrifuged at 3,000 rpm for 15 minutes, and serum was separated therefrom and used as samples for AST and ALT analysis. The results are shown in Table 4 below and FIG. 8.

**[Table 4]**

| | CON | CCL₄ | Comparative Example 1 | Example 1 |
|---|---|---|---|---|
| AST (IU/L) | 105.3 | 573.4 | 416.0 | 321.5 |
| ALT (IU/L) | 35.6 | 547.3 | 391.8 | 306.7 |

As shown in Table 4 above and FIG. 8, it was confirmed that the AST and ALT levels tended to decrease in the group treated with the *Agrimonia eupatoria* extract powder (Comparative Example 1) compared to the CCL group, and in particular, were significantly low in the group treated with the *Agrimonia eupatoria* inclusion extract powder (Example 1).

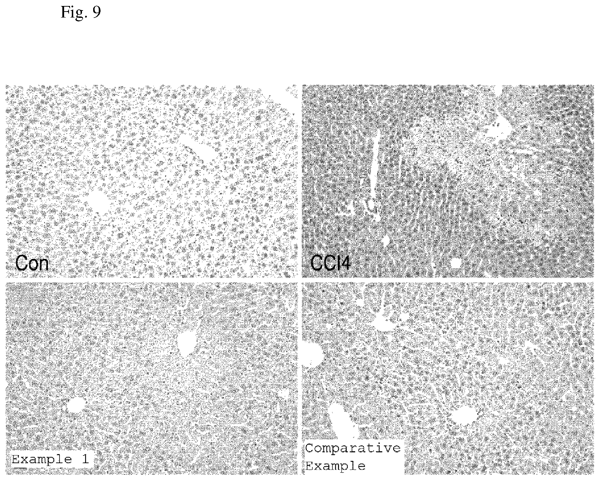

Liver histopathological analysis was performed to examine the acute hepatotoxicity preventive effect and antioxidant effect of each of the *Agrimonia eupatoria* extract powder and inclusion extract powder, and the results are shown in FIG. 9.

As shown in FIG. 9, as a result of performing histopathological analysis of the experimental animals of each group, rapid changes in liver injury, including cell disruption and bile duct proliferation, could be observed in the liver tissue of the control group (CC14) treated with carbon tetrachloride (CC14) alone. On the other hand, some injury appeared in the group treated with the *Agrimonia eupatoria* extract powder (Comparative Example 1), but was similar to that in the control group, and in particular, the group treated with the *Agrimonia eupatoria* inclusion extract powder (Example 1) showed almost the same tissue findings as the control group.

## Claims

1. A method for preparing an *Agrimonia eupatoria* extract comprising step a) of obtaining an extract from *Agrimonia eupatoria* using an extraction solvent containing cyclodextrin.

2. The method of claim 1, wherein step a) comprises:
step a-1) of preparing an extraction solvent by adding cyclodextrin to an organic solvent; and
step a-2) of obtaining the extract by adding *Agrimonia eupatoria* to the extraction solvent.

3. The method of claim 1, wherein step a-1) comprises preparing the extraction solvent by heating at 20 to 100°C for 3 to 8 hours after adding cyclodextrin to the organic solvent.

4. The method of claim 2, wherein the organic solvent in step a-1) is at least one selected from the group consisting of methanol, ethanol, n-propanol, iso-propanol, n-butanol, iso-butanol, 2-butanol, and 3-butanol.

5. The method of claim 2, wherein the cyclodextrin in step a-1) is at least one selected from the group consisting of alpha-cyclodextrin, beta-cyclodextrin, and gamma-cyclodextrin.

6. The method of claim 2, wherein the cyclodextrin in step a-1) is used in an amount of 0.1 to 40 parts by weight based on the total weight of *Agrimonia eupatoria* added in step a-2)

7. The method of claim 2, wherein step a-2) comprises obtaining the extract by extraction at 20 to 100°C for 1 to 40 hours after adding *Agrimonia eupatoria* to the extraction solvent.

8. The method of claim 2, wherein *Agrimonia eupatoria* in step a-2) is used in an amount of 0.01 to 50 parts by weight based on the total weight of the extraction solvent.

9. The method of claim 2, wherein the extract in step a-2) contains a sparingly soluble component.

10. The method of claim 2, wherein the extract in step a-2) contains 0.1 to 5% by weight of luteolin-7-O-glucuronide, which is an indicator active ingredient and forms an inclusion complex.

11. The method of claim 1, further comprising, after step a), step b) of filtering the extract.

12. The method of claim 11, further comprising, after step b), step b') of obtaining an extract from filtration residue using an organic solvent containing cyclodextrin.

13. A food composition for improving liver function containing an extract prepared by the method of any one of claims 1 to 12.

14. A pharmaceutical composition for treating liver disease containing an extract prepared by the method of any one of claims 1 to 12.
